# EUROPEAN PATENT APPLICATION

(11) **EP 1 591 528 A2**
(43) Date of publication of application: **02.11.2005**
(21) Application number: 05013342.0
(22) Date of filing: 16.10.1997
(51) Int. Cl.: C12N 15/21, A61K 48/00, C07K 14/56, C12N 15/86

(54) **Method and compositions for delivery and expression of interferon-alpha nucleic acids**

(30) Priority: 18.10.1996 US 733815
(62) Divisional of application: 97910794.3
(71) Applicant: CANJI, Inc., San Diego, CA 92121 (US)
(72) Inventor: Nagabhusham, Tattanahalli L., Parsippany NJ 07054 (US); Saha, Deba P., Nutley NJ 07110 (US)
(74) Representative: UEXKÜLL & STOLBERG

(57) **Abstract**

Methods and compositions are provided for the tissue-specific expression of interferon alpha for therapeutic purposes.

## Description

### BACKGROUND OF THE INVENTION

The human interferon alphas (IFN-α) are a family of proteins comprising at least 24 subspecies (Zoon, K.C., Interferon **9:1** (1987), Gresser, I., ed., Academic Press, NY). The interferon alphas were originally described as agents capable of inducing an antiviral state in cells but are now known as pleiotropic lymphokines affecting many functions of the immune system (Openakker et al. Experimentia **45:513** (1989)).

IFN-α has been widely used for therapeutic purposes, including hairy cell leukemia, kaposi's sarcoma, renal cell carcinoma, non Hodgkin's lymphoma, T-cell leukemia, multiple and chronic myelogenous leukemia, malignant melanoma, bladder cell carcinoma, colon carcinoma (with 5-FU), condyloma acuminata, rhinovirus and various forms of chronic viral hepatitis occurring as a result of hepatitis B virus.(HBV), hepatitis C virus (HCV), non A non B virus (NANB), or hepatitis δ virus (HDV) infection (Pestka AIDA Research & Human Retroviruses **8(5):776-786** (1992)). IFN-α has also been found to be highly effective against megakaryocytopoiesis and controlling thrombocytosis in patients with myeloproliferative disorders (Talpaz et al. Annals Int. Med. **99:789-792** (1983); Gisslinger et al. Lancet **i:634-637** (1989); Ganser et al. Blood **70:1173-1179** (1987)).

Gene therapy techniques have the potential for limiting the exposure of a subject to a gene product, such as interferon, by targeting the expression of the therapeutic gene to a tissue of interest. However, in general, the ability to target the tissue of interest is one of the major challenges of gene therapy. As an example of the targeting of interferon genes, WIPO Patent Application Publication No. WO 93/15609 discloses the delivery of interferon genes to vascular tissue by administration of such genes to areas of vessel wall injury using a catheter system. In another example, an adenoviral vector encoding a protein capable of enzymatically converting a prodrug, a "suicide gene", and a gene encoding a cytokine are administered directly into a solid tumor.

Other methods of targeting therapeutic genes to tissues of interest include the three general categories of transductional targeting, positional targeting, and transcriptional targeting (for a review, see, e.g., Miller et al. FASEB J. **9:190-199** (1995)). Transductional targeting refers to the selective entry into specific cells, achieved primarily by selection of a receptor ligand. Positional targeting within the genome refers to integration into desirable loci, such as active regions of chromatin, or through homologous recombination with an endogenous hucleotide sequence such as a target gene. Transcriptional targeting refers to selective expression attained by the incorporation of transcriptional promoters with highly specific regulation of gene expression tailored to the cells of interest.

Examples of tissue-specific promoters include the promoter for creatine kinase, which has been used to direct the expression of dystrophin cDNA expression in muscle and cardiac tissue (Cox et al. Nature **364:725-729** (1993)); and immunoglobulin heavy or light chain promoters for the expression of suicide genes in B cells (Maxwell et al. Cancer Res. **51:4299-4304** (1991)). An endothelial cell-specific regulatory region has also been characterized (Jahroudi et al. Mol. Cell. Biol. **14:999-1008** (1994)). Amphotrophic retroviral vectors have been constructed carrying a herpes simplex virus thymidine kinase gene under the control of either the albumin or alpha-fetoprotein promoters (Huber et al. Proc. Natl. Acad. Sci. U.S.A. **88:8039-8043** (1991)) to target cells of liver lineage and hepatoma cells, respectively. Such tissue specific promoters can be used in retroviral vectors (Hartzoglou et al.. J. Biol. Chem. **265:17285-17293** (1990)) and adenovirus vectors (Friedman et al. Mol. Cell. Biol. **6:3791-3797** (1986)) and still retain their tissue specificity.

Thus, there is a need for targeting expression of alpha interferon for the treatment of cancer, hepatitis, and other conditions amenable to therapy with alpha interferon. The instant invention addresses this need, and more.

### SUMMARY OF THE INVENTION

One aspect of the invention is a method for providing a patient with an interferon alpha polypeptide comprising introducing into.a tissue of interest of the patient a vector comprising a nucleic acid segment encoding an interferon alpha polypeptide, the nucleic acid segment being operatively linked to a promoter having specificity for the tissue of interest, wherein the polypeptide is expressed in the tissue of interest.

Another aspect of the invention is a method for increasing interferon alpha levels in a tissue of interest in a patient comprising introducing into the tissue of interest a vector comprising a nucleic acid segment encoding an interferon alpha polypeptide, the nucleic acid segment being operatively linked to a promoter having specificity for the tissue of interest, wherein the interferon alpha polypeptide is expressed in the tissue of interest in the patient.

Another aspect of the invention is a method for treatment of cancer responsive to interferon alpha comprising administering to a cancerous tissue a vector comprising a nucleic acid segment encoding an interferon alpha polypeptide, the nucleic acid segment encoding an interferon alpha polypeptide, the nucleic acid segment being operatively linked to a promoter having specificity for the tissue, wherein the alpha interferon polypeptide is expressed in the tissue.

Another aspect of the invention is a method of treatment of hepatitis comprising administering to a patient's liver a vector comprising a nucleic acid segment encoding an interferon alpha polypeptide, the nucleic acid segment being operatively linked to a promoter having specificity for liver cells, wherein the interferon alpha polypeptide is expressed in the patient's liver.

A further aspect of the invention is a composition comprising a vector comprising a nucleic acid segment encoding an interferon alpha polypeptide, the nucleic acid segment being operatively linked to a promoter having specificity for a tissue of interest.

### BRIEF DESCRIPTION OF THE FIGURES

Figure 1 is a graph depicting the antiproliferative effects of interferon alpha on human prostate cancer cells.
Figure 2 is a graph depicting luciferase activity as a measure of luciferase expression driven by promoters of liver-specific genes.

### DETAILED DESCRIPTION OF THE SPECIFIC EMBODIMENT

The instant invention provides methods for the tissue specific expression of IFN-α using tissue specific promoters. The term IFN-α as used herein is intended to include all subclasses of interferon alpha, deletion, insertion, or substitution variants thereof, biologically active fragments, and allelic forms. "Biologically active " as used herein refers to any anti-viral or anti-proliferative activity as measured by techniques well known in the art (see, for example, Openakker et al., supra; Mossman J. Immunol. Methods **65:55** (1983)). Recombinant interferon alphas have been cloned and expressed in *E. coli* by several groups (for example, Weissmann et al. Science **209:1343-1349** (1980); Sreuli et al. Science **209:1343-1347** (1980); Goeddel et al. Nature **290:20-26** (1981); Henco et al. J. Mol. Biol. **185:227-260** (1985)). Preferably, the interferon alpha is interferon alpha 2a or 2b (see, for example, WO 91/18927), although any interferon alpha may be used.

Nucleic acids encoding the IFN-α polypeptide can be DNA or RNA. The phrase "nucleic acid sequence encoding" refers to a nucleic acid which directs the expression of a specific protein or peptide. The nucleic acid sequences include both the DNA strand sequence that is transcribed into RNA and the RNA sequence that is translated into protein. The nucleic acid sequences include both the full length nucleic acid sequences as well as non-full length sequences derived from the full length protein. It is further understood that the sequence includes the degenerate codons of the native sequence or sequences which may be introduced to provide codon preference in a specific host cell.

The term "vector", refers to viral expression systems, autonomous self-replicating circular DNA (plasmids), and includes both expression and nonexpression plasmids. Where a recombinant microorganism or cell culture is described as hosting an "expression vector," this includes both extrachromosomal circular DNA and DNA that has been incorporated into the host chromosome(s). Where a vector is being maintained by a host cell, the vector may either be stably replicated by the cells during mitosis as an autonomous structure, or is incorporated within the host's genome. A vector contains multiple genetic elements positionally and sequentially oriented, i.e., operatively linked with other necessary elements such that nucleic acid in the vector encoding IFN-α can be transcribed, and when necessary, translated in transfected cells.

The term "gene" as used herein is intended to refer to a nucleic acid sequence which encodes a polypeptide. This definition includes various sequence polymorphisms, mutations, and/or sequence variants wherein such alterations do not affect the function of the gene product. The term "gene" is intended to include not only coding sequences but also regulatory regions such as promoters, enhancers, and termination regions. The term further includes all introns and other DNA sequences spliced from the mRNA transcript, along with variants resulting from alternative splice sites.

The term "plasmid" refers to an autonomous circular DNA molecule capable of replication in a cell, and includes both the expression and nonexpression types. Where a recombinant microorganism or cell culture is described as hosting an "expression plasmid", this includes both extrachromosomal circular DNA molecules and DNA that has been incorporated into the host chromosome(s). Where a plasmid is being maintained by a host cell, the plasmid is either being stably replicated by the cells during mitosis as an autonomous structure or is incorporated within the host's genome.

The phrase "recombinant protein" or "recombinantly produced protein" refers to a peptide or protein produced using non-native cells that do not have an endogenous copy of DNA able to express the protein. The cells produce the protein because they have been genetically altered by the introduction of the appropriate nucleic acid sequence. The recombinant protein will not be found in association with proteins and other subcellular components normally associated with the cells producing the protein. The terms "protein" and "polypeptide" are used interchangeably herein.

In general, the IFN-α is provided in an expression vector comprising the following elements linked sequentially at appropriate distances for functional expression: a tissue-specific promoter, an initiation site for transcription, a 3' untranslated region, a 5' mRNA leader sequence, a nucleic acid sequence encoding an alpha interferon polypeptide, and a polyadenylation signal. Enhancer sequences and other sequences aiding expression and/or secretion can also be included in the expression vector. Additional genes, such as those encoding drug resistance, can be included to allow selection or screening for the presence of the recombinant vector. Such additional genes can include, for example, genes encoding neomycin resistance, multi-drug resistance, thymidine kinase, beta-galactosidase, dihydrofolate reductase (DHFR), and chloramphenicol acetyl transferase.

In the instant invention, targeting of the IFN-α to a particular tissue of interest is accomplished by the use of a promoter and/or other expression elements preferentially used by the tissue of interest. Examples of known tissue-specific promoters include the promoter for creatine kinase, which has been used to direct the expression of dystrophin cDNA expression in muscle and cardiac tissue (Cox et al. Nature **364:725-729** (1993)); immunoglobulin heavy or light chain promoters for the expression of genes in B cells; albumin or alpha-fetoprotein promoters to target cells of liver lineage and hepatoma cells, respectively.

Exemplary tissue-specific expression elements for the liver include but are not limited to HMG-CoA reductase promoter (Luskey, Mol. Cell. Biol. **7(5):1881-1893** (1987)); sterol regulatory element 1 (SRE-1; Smith et al. J. Biol. Chem. **265(4):2306-2310** (1990); phosphoenol pyruvate carboxy kinase (PEPCK) promoter (Eisenberger et al. Mol. Cell Biol. **12(3):1396-1403** (1992)); human C-reactive protein (CRP) promoter (Li et al. J. Biol. Chem. **265(7):4136-4142** (1990)); human glucokinase promoter (Tanizawa et al. Mol. Endocrinology **6(7):1070-81** (1992); cholesterol 7-alpha hydroylase (CYP-7) promoter (Lee et al. J. Biol. Chem. **269(20):14681-9** (1994)); beta-galactosidase alpha-2,6 sialyltransferase promoter (Svensson et al. J. Biol. Chem. **265(34):20863-8** (1990); insulin-like growth factor binding protein (IGFBP-1) promoter (Babajko et al. Biochem Biophys. Res. Comm. **196 (1):480-6** (1993)); aldolase B promoter (Bingle et al. Biochem J. **294(Pt2):473-9** (1993)); human transferrin promoter (Mendelzon et al. Nucl. Acids Res. **18(19):5717-21** (1990); collagen type I promoter (Houglum et al. J. Clin. Invest. **94(2):808-14** (1994)).

Exemplary tissue-specific expression elements for the prostate include but are not limited to the prostatic acid phosphatase (PAP) promoter (Banas et al. Biochim. Biophys. Acta. **1217(2):188-94** (1994); prostatic secretory protein of 94 (PSP 94) promoter (Nolet et al. Biochim. Biophys. ACTA **1098(2):247** **9** (1991)); prostate specific antigen complex promoter (Casper et al. J. Steroid Biochem. Mol. Biol. **47 (1-6) :127-35** (1993)); human glandular kallikrein gene promoter (hgt-1) (Lilja et al. World J. Urology **11(4):188-91** (1993).

Exemplary tissue-specific expression elements for gastric tissue include but are not limited to the human H⁺/K⁺-ATPase alpha subunit promoter (Tanura et al. FEBS Letters **298: (2-3):137-41** (1992)).

Exemplary tissue-specific expression elements for the pancreas include but are not limited to pancreatitis associated protein promoter (PAP) (Dusetti et al. J. Biol. Chem. **268(19):14470-5** (1993)); elastase 1 transcriptional enhancer (Kruse et al. Genes and Development **7(5):774-86** (1993)); pancreas specific amylase and elastase enhancer promoter (Wu et al. Mol. Cell. Biol. **11(9):4423-30** (1991); Keller et al. Genes & Dev. **4(8):1316-21** (1990)); pancreatic cholesterol esterase gene promoter (Fontaine et al. Biochemistry **30(28) :7008-14** (1991)).

Exemplary tissue-specific expression elements for the endometrium include but are not limited to the uteroglobin promoter (Helftenbein et al. Annal. NY Acad. Sci. **622:69-79** (1991)).

Exemplary tissue-specific expression elements for adrenal cells include but are not limited to cholesterol side-chain cleavage (SCC) promoter (Rice et al. J. Biol. Chem. **265:11713-20** (1990).

Exemplary tissue-specific expression elements for the general nervous system include but are not limited to gamma-gamma enolase (neuron-specific enolase, NSE) promoter (Forss-Petter et al. Neuron **5(2):187-97** (1990)).

Exemplary tissue-specific expression elements for the brain include but are not limited to the neurofilament heavy chain (NF-H) promoter (Schwartz et al. J. Biol. Chem, **269(18):13444-50** (1994)).

Exemplary tissue-specific expression elements for lymphocytes include but are not limited to the human CGL-1/granzyme B promoter (Hanson et al. J. Biol. Chem. **266 (36):24433-8** (1991)); the terminal deoxy transferase (TdT), lambda 5, VpreB, and lck (lymphocyte specific tyrosine protein kinase p561ck) promoter (Lo et al. Mol. Cell. Biol. **11(10):5229-43** (1991)); the humans CD2 promoter and its 3 transcriptional enhancer (Lake et al. EMBO J . **9 (10):3129-36** (1990)), and the human NK and T cell specific activation (NKG5) promoter (Houchins et al. Immunogenetics **37(2):102-7** (1993)).

Exemplary tissue-specific expression elements for the colon include but are not limited to pp60c-src tyrosine kinase promoter (Talamonti et al. J. Clin. Invest **91(1) :53-60** (1993)); organ-specific neoantigens (OSNs), mw 40kDa (p40) promoter (Ilantzis et al. Microbiol. Immunol. **37(2):119-28** (1993)); colon specific antigen-P promoter (Sharkey et al. Cancer **73 (3 supp.) 864-77** (1994)).

Exemplary tissue-specific expression elements for breast cells include but are not limited to the human alpha-lact.albumin promoter (Thean et al. British J. Cancer. **61(5) :773-5** (1990)).

Other elements aiding specificity of expression in a tissue of interest can include secretion leader sequences, enhancers, nuclear localization signals, endosmolytic peptides, etc. Preferably,' these elements are derived from the tissue of interest to aid specificity.

Techniques for nucleic acid manipulation of the nucleic acid sequences of the invention such as subcloning nucleic acid sequences encoding polypeptides into expression vectors, labelling probes, DNA hybridization, and the like are described generally in Sambrook *et al*., Molecular Cloning - A Laboratory Manual (2nd Ed.), Vol. 1-3, Cold Spring Harbor Laboratory, Cold Spring Harbor, New York, (1989), which is incorporated herein by reference. This manual is hereinafter referred to as "Sambrook *et al*."

Once DNA encoding a sequence of interest is isolated and cloned, one can express the encoded proteins in a variety of recombinantly engineered cells. It is expected that those of skill in the art are knowledgeable in the numerous expression systems available for expression of DNA encoding. No attempt to describe in detail the various methods known for the expression of proteins in prokaryotes or eukaryotes is made here.

In brief summary, the expression of natural or synthetic nucleic acids encoding a sequence of interest will typically be achieved by operably linking the DNA or cDNA to a promoter (which is either constitutive or inducible), followed by incorporation into an expression vector. The vectors can be suitable for replication and integration in either prokaryotes or eukaryotes. Typical expression vectors contain transcription and translation terminators, initiation sequences, and promoters useful for regulation of the expression of polynucleotide sequence of interest. To obtain high level expression of a cloned gene, it is desirable to construct expression plasmids which contain, at the minimum, a strong promoter to direct transcription, a ribosome binding site for translational initiation, and a transcription/translation terminator. The expression vectors may also comprise generic expression cassettes containing at least one independent terminator sequence, sequences permitting replication of the plasmid in both eukaryotes and prokaryotes, i.e., shuttle vectors, and selection markers for both prokaryotic and eukaryotic systems. See Sambrook *et al*.

The constructs of the invention can be introduced into the tissue of interest in vivo or ex vivo by a variety of methods. In some embodiments of the invention, the vector is introduced to cells by such methods as microinjection, calcium phosphate precipitation, liposome fusion, or biolistics. In further embodiments, the DNA is taken up directly by the tissue of interest. In other embodiments, the constructs are packaged into a viral vector system to facilitate introduction into cells.

Viral vector systems useful in the practice of the instant invention include adenovirus, herpesvirus, adeno-associated virus, minute virus of mice (MVM), HIV, sindbis virus, and retroviruses such as Rous sarcoma virus, and MoMLV. Typically, the constructs of the instant invention are inserted into such vectors to allow packaging of the interferon expression construct, typically with accompanying viral DNA, infection of a sensitive host cell, and expression of the interferon-α gene. A particularly advantageous vector is the adenovirus vector disclosed by Wills et al. Hum. Gene Therapy **5:1079-1088** (1994).

In still other embodiments of the invention, the recombinant IFN-α constructs of the invention are conjugated to a cell receptor ligand for facilitated uptake (e.g., invagination of coated pits and internalization of the endosome) through a DNA linking moiety (Wu et al. J; Biol. Chem. **263:14621-14624** (1988); WO 92/06180). For example, the DNA constructs of the invention can be linked through a polylysine moiety to asialo-oromucocid, which is a ligand for the asialoglycoprotein receptor of hepatocytes.

Similarly, viral envelopes used for packaging the constructs of the invention can be modified by the addition of receptor ligands or antibodies specific for a receptor to permit receptor-mediated endocytosis into specific cells (e.g., WO 93/20221, WO 93/14188; WO 94/06923). In some embodiments of the invention, the DNA constructs of the invention are linked to viral proteins, such as adenovirus particles, to facilitate endocytosis (Curiel et al. Proc. Natl. Acad. Sci. U.S.A. **88:8850-8854** (1991)). In other embodiments, molecular conjugates of the instant invention can include microtubule inhibitors (WO/9406922); synthetic peptides mimicking influenza virus hemagglutinin (Plank et al. J. Biol. Chem. **269:12918-12924** (1994)); and nuclear localization signals such as SV40 T antigen (WO93/19768).

The term "treatment" as used herein is intended to refer to the introduction of nucleic acid encoding an alpha interferon to a patient for the purpose of exposing a tissue of interest, especially a tissue having one or more cells demonstrating some pathology, to alpha interferon. Thus, for example, a "cancerous" tissue is intended to refer to a tissue in which one or more cells is classified as cancerous, malignant, tumorous, precancerous, transformed, or as an adenoma or carcinoma, or any other synonym commonly used in the art for these conditions.

A "noncancerous" cell as used herein is understood in the art as excluded from the definition of cancerous or cancer cell, and can include normal cells and cells displaying some pathological feature such as infection by a virus, bacterium, parasite, or other organism, cells affected by a hereditable condition that renders them less optimal than normal or wild type counterparts, cells affected by some presumed non-infectious disease state such as diabetes, etc., and cells which have survived any of these stresses, etc.

Treatment or therapy of any condition which would benefit from administration of IFN-α can begin prior to the diagnosis of the condition or at any time after diagnosis of a condition. Thus, for example, a patient suspected of having a precancerous lesion or an increased probability of developing some type of cancer can be treated with the compositions of the invention. Similarly, a person exposed to a pathogen, such as hepatitis B virus, can be treated with the compositions of the invention before hepatitis is diagnosed. Furthermore, suspected carriers of HBV or patients likely to become carriers can be treated after gross symptoms of the disease have improved.

The constructs of the invention are useful in the therapy of various cancers, hepatitis and other conditions in which the administration of IFN-α to raise IFN-α levels in tissues is advantageous, including but not limited to ulcerative colitis, rhinovirus infections, condyloma acuminata, laryngeal papillomitis; HIV infection, fibrosis, allergic diseases due to excess IL-4 and IgE production, and granulomatous disorders, such as Crohn's disease. Although any tissue can be targeted for which some tissue-specific expression element, such as a promoter, can be identified, of particular interest is the tissue specific administration of IFN-α to raise IFN-α levels in cancerous tissues, such as human prostate carcinoma and hepatoma tissues. Furthermore, the constructs of the invention can be used to raise IFN-α levels in tissues in pathological conditions in which non-cancerous cells are deficient in interferon production, i.e,, produce less interferon than the healthy cells, such as in chronic hepatitis B virus carriers (Nouri-Aria et al. Hepatology **14(6):1308-1311** (1991)). In some embodiments of the invention the recombinant constructs are targeted to neighboring tissues or cells to raise the local concentration of interferon alpha in a cell population of interest.

The compositions of the invention will be formulated for administration by manners known in the art acceptable for administration to a mammalian subject, preferably a human. In some embodiments of the invention, the compositions of the invention can be administered directly into a tissue by injection or into a blood vessel supplying the tissue of interest. In further embodiments of the invention the compositions of the invention are administered "locoregionally", i.e., intravesically, intralesionally, and/or topically. In other embodiments of the invention, the compositions of the invention are administered systemically by injection, inhalation, suppository, trarisdermal delivery, etc. In further embodiments of the invention, the compositions are administered through catheters or other devices to allow access to a remote tissue of interest, such as an internal organ. The compositions of the invention can also be administered in depot type devices, implants, or encapsulated formulations to allow slow or sustained release of the compositions.

The invention provides compositions for administration which comprise a solution of the compositions of the invention dissolved or suspended in an acceptable carrier, preferably an aqueous carrier. A variety of aqueous carriers may be used, e.g., water, buffered water, 0.8% saline, 0.3% glycine, hyaluronic acid and the like. These compositions may be sterilized by conventional, well known sterilization techniques, or may be sterile filtered. The resulting aqueous solutions may be packaged for use as is, or lyophilized, the lyophilized preparation being combined with a sterile solution prior to administration. The compositions may contain pharmaceutically acceptable auxiliary substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, etc.

The concentration of the compositions of the invention in the pharmaceutical formulations can vary widely, i.e., from less than about 0.1%, usually at or at least about 2% to as much as 20% to 50% or more by weight, and will be selected primarily by fluid volumes, viscosities, etc., in accordance with the particular mode of administration selected.

The compositions of the invention may also be administered via liposomes. Liposomes include emulsions, foams, micelles, insoluble monolayers, liquid crystals, phospholipid dispersions, lamellar layers and the like. In these preparations the composition of the invention to be delivered is incorporated as part of a liposome, alone or in conjunction with a molecule which binds to a desired target, such as antibody, or with other therapeutic or immunogenic compositions. Thus, liposomes either filled or decorated with a desired composition of the invention of the invention can delivered systemically, or can be directed to a tissue of interest, where the liposomes then deliver the selected therapeutic/immunogenic peptide compositions.

Liposomes for use in the invention are formed from standard vesicle-forming lipids, which generally include neutral and negatively charged phospholipids and a sterol, such as cholesterol. The selection of lipids is generally guided by consideration of, e.g., liposome size, acid lability and stability of the liposomes in the blood stream. A variety of methods are available for preparing liposomes, as described in, e.g., Szoka et al. Ann. Rev. Biophys. Bioeng. **9:467** (1980), U.S. Patent Nos. 4,235,871, 4,501,728, 4,837,028, and 5,019,369, incorporated herein by reference.

A liposome suspension containing a composition of the invention may be administered intravenously, locally, topically, etc. in a dose which varies according to, inter alia, the manner of administration, the composition of the invention being delivered, and the stage of the disease being treated.

For solid compositions, conventional nontoxic solid carriers may be used which include, for example, pharmaceutical grades of mannitol, lactose, starch, magnesium stearate, sodium saccharin, talcum, cellulose, glucose, sucrose, magnesium carbonate, and the like. For oral administration, a pharmaceutically acceptable nontoxic composition is formed by incorporating any of the normally employed excipients, such as those carriers previously listed, and generally 10-95% of active ingredient, that is, one or more compositions of the invention of the invention, and more preferably at a concentration of 25%-75%.

For aerosol administration, the compositions of the invention are preferably supplied in finely divided form along with a surfactant and propellant. Typical percentages of compositions of the invention are 0.01%-20% by weight, preferably 1%-10%. The surfactant must, of course, be nontoxic, and preferably soluble in the propellant. Representative of such agents are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride. Mixed esters, such as mixed or natural glycerides may be employed. The surfactant may constitute 0.1%-20% by weight of the composition, preferably 0.25-5%. The balance of the composition is ordinarily propellant. A carrier can also be included, as desired, as with, e.g., lecithin for intranasal delivery.

The constructs of the invention can additionally be delivered in a depot-type system, an encapsulated form, or an implant by techniques well-known in the art. Similarly, the constructs can be delivered via a pump to a tissue of interest.

In some embodiments of the invention, the compositions of the invention are administered ex vivo to cells or tissues explanted from a patient, then returned to the patient. Examples of ex vivo administration of gene therapy constructs include Arteaga et al. Cancer Research **56(5):1098-1103** (1996); Nolta et al. Proc Natl. Acad. Sci. USA **93(6):2414-9** (1996); Koc et al. Seminars in Oncology **23 (1):46-65** (1996); Raper et al. Annals of Surgery **223(2) :116-26** (1996); Dalesandro et al. J. Thorac. Cardi. Surg. **11(2):416-22** (1996); and Makarov et al. Proc. Natl. Acad. Sci. USA **93(1):402-6** (1996).

The following examples are included for illustrative purposes and should not be considered to limit the present invention.

### EXAMPLES

### I. Effective Interferon-alpha on Prostate Cancer Cell Proliferation

Three different prostate carcinoma cells, LNCaP (androgen dependent, ATCC #CRL 1740), PC-3 cells (androgen independent, ATCC #CRL 1435), and DU-145 (androgen independent, ATCC #HTB 81) were studied. The cells were grown in 5 different concentrations (10, 10², 10³, 10⁴, 10⁵ IU/ml) of interferon-α2b (Schering-Plough) for 72 hours in the following media: PC-3 was cultured in Ham's F12 K medium (GIBCO BRL) supplemented with 7% fetal bovine serum; DU-145 was cultured in DMEM (GIBCO BRL) supplemented with 10% fetal calf serum; LnCaP was cultured in RPMI 1640 (GIBCO BRL ) supplemented with 5% fetal bovine serum.

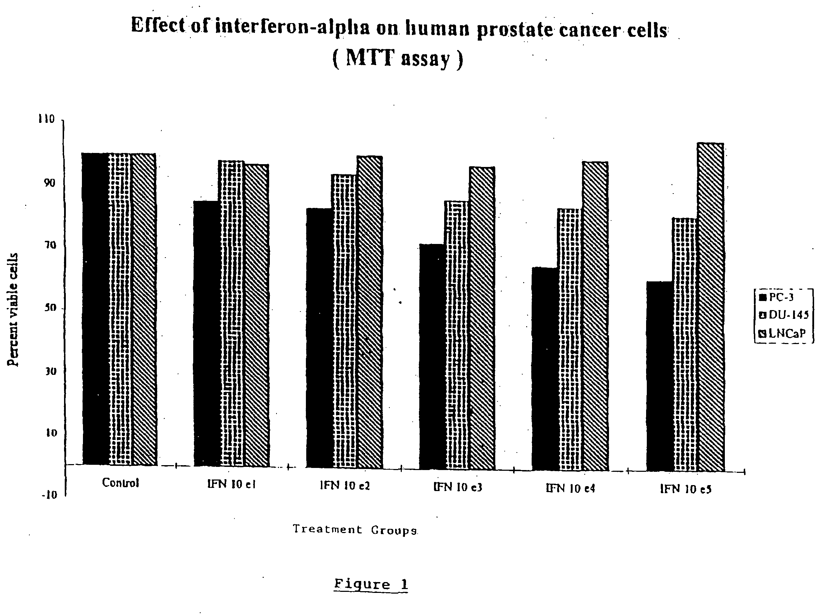

Antiproliferative effects of interferon were measured by MTT assay (Mossman J. Immunol. Methods **65:55** (1983)). PC-3 and DU-145 cells showed a consistent sensitivity to increasing concentrations of interferon plateauing at 10⁴ IU/ml. Androgen sensitive LNCaP cells did not respond to IFN. Between the two androgen refractory cells, PC-3 appeared more sensitive than DU-145 cells. These data are summarized in Figure 1. Solid bars represent cell line PC-3 (androgen independent); cross-hatched bars represent cell line DU-145 (androgen independent); diagonal bars represent LNCaP (androgen dependent).

### II. Construction of an Expression Cassette

An expression vector was constructed having the complete cDNA sequence of interferon alpha 2b (IFN-α2b) and the complete signal sequence for IFN-α2b (Sreuli et al. Science **209:1343-1347** (1980); Goeddel et al. Nature **290:20-26** (1981); Henco et al. J. Mol. Biol. **185:227-260** (1985)) under control of an approximately 600 bp basal promoter for the prostate specific antigen gene (PSA) for tissue specific expression of IFN-α in prostate carcinoma cells. Basically, full-length IFN-α2b cDNA having its putative signal leader at the 5' end was cloned into the polycloning site at HindII and Eco RI downstream of the CMV promoter in the mammalian expression vector PCDNA3 (Invitrogen) to create plasmid DIFN. The 5' flanking sequence of the PSA gene containing the PSA promoter (BBRC **161:1151-1159** (1989); Genebank #M27274) was inserted into the vector, replacing the CMV promoter, to create plasmid PSADIFN.

### III. Cloning of basal promoters for liver specific genes

5' flanking sequences, including basal promoters, from four human liver specific genes, albumin (HAL), α1-antitrypsin (HAT), alpha feto protein (AFP), and hydroxy-methyl-glutaryl CoA reductase (HMG), were subcloned from ATCC 65731, ATCC 61597, ATCC 65735, and ATCC 59567, respectively, into pCRScript vector for use in interferon gene delivery and its tissue specific expression in hepatic cells (Luskey Mol. Cell. Biol. **7(5):1881-1893** (1987); Minghetti et al. J. Biol. Chem. **261(15):6747-6757** (1986); Long et al. Biochemistry **23:4828-4837** (1984); Gibbs Biochemistry **26:1332-1343** (1987)).

After restriction enzyme mapping of the inserts in the pCRScript vector containing 5'-flanking sequences of the above genes for liver specific enzymes, the inserts were placed upstream of luciferase gene in the reporter plasmid, pGL3 (Promega) from pCRScript vector. Chinese hamster ovary (CHO-K1, ATCC # CCL-61), human hepatoma (HepG2, ATCC #NB 8065) and human hepatoma (Hep3B ATCC # HB 8064) cells were transfected by electroporation with these four constructs as well as by the control plasmid pGLC (PROMEGA Corp). pGLC contains the luciferase gene under the control of the SV40 promoter and the SV40 enhancer.

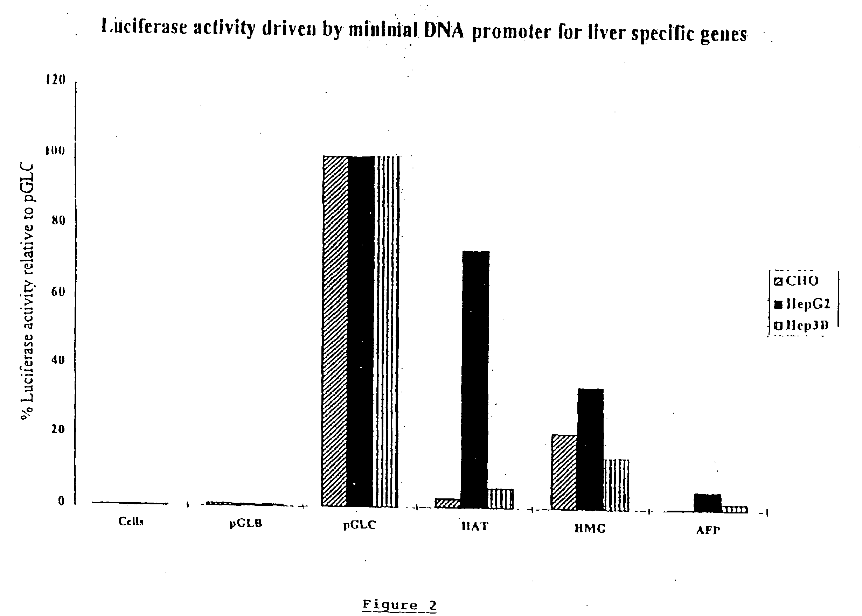

Luciferase expression by the four liver specific promoter sequences in the transfected cells was compared with the control plasmid pGLC and the vector pGL3. The data are summarized in Figure 2 (HAL result not shown). Three cell types were transfected by DNA constructs having the salient features indicated. Solid bars represent human hepatoma HepG2 cells; cross-hatched bars represent Chinese hamster ovary cells (CHO); diagonal bars represent human hepatoma Hep3B cells (Hep3B). pGLB is a negative control plasmid; pGLC is a positive control plasmid; HAT denotes the human α1-antitrypsin promoter; HMG denotes the human hydroxy-methyl-glutaryl CoA reductase promoter; AFP denotes the human α-feto protein promoter. Of the four tissue-specific promoters, the human α1-antitrypsin (HAT) promoter appeared to be the best candidate for liver-specific expression under these conditions.

Expression driven by the HAT promoter can be further optimized by constructing an expression vector with a liver specific enhancer sequence such as the human α-fetoprotein enhancer (Watanabe, et al. J. Biol. Chem. **262(10):4812-4818** (1987), Genebank # J02693), the human albumin enhancer (Hayashi et al. J. Biol. Chem. **267(21):14580-14585** (1992), Genebank # N92816), the human α-1 microglobulin/bikunin enhancer (Rouet et al. J. Biol. Chem. **267(29):20765-20773** (1992), Genebank # X67082), or the hepatitis B enhancer (Valenzuela et al. Animal Virus Genetics ed. B. Biels et al., **p.p. 57-70,** Academic Press, N.Y. (1981); Galibert et al. Nature **281:646-650** (1979)).

Although the foregoing invention has been described in some detail by way of illustration and example for purposes of clarity of understanding, it will be obvious that certain changes and modification may be practiced within the scope of the appended claims.

All references cited herein are incorporated by reference in their entirety for all purposes.

## Claims

**1.** A recombinant adenoviral vector for expression of an interferon alpha polypeptide, said adenoviral vector comprising a nucleic acid sequence encoding an interferon alpha polypeptide operably linked to a promoter functional in a mammalian cell;
wherein said interferon alpha polypeptide is interferon alpha 2b.

**2.** The recombinant vector of claim 1 wherein said adenoviral vector is a adenoviral vector disclosed in Wills et al., Human Gene Therapy 5: 1079-1088 (1994).

**3.** The recombinant vector of claims 1 or 2 wherein said nucleic acid sequence encoding an interferon alpha polypeptide is operably linked to a nucleic acid encoding an interferon alpha secretion leader

**4.** The recombinant vector of claim 1 wherein the promoter is a constitutive promoter.

**6.** A pharmaceutical formulation comprising as an active ingredient a vector according to claims 1 to 4 associated with one or more pharmaceutically acceptable carriers, diluents or excipients therefore.

**6.** Use of a vector as claimed in claims 1 to 4 for preparing a pharmaceutical for treating a mammal suffering from cancer.

**7.** Use of claim 6, wherein the pharmaceutical is for intravesical administration.

**8.** Use of a vector as claimed in claims 1 to 4 for preparing a pharmaceutical for treating hepatoma.

**9.** A composition comprising a vector, said vector comprising a nucleic acid segment encoding an interferon alpha polypeptide, the nucleic acid segment being operatively linked to a promoter specific for a tissue of interest.

**10.** The composition of claim 9, wherein the vector is viral.

**11.** The composition of claim 10, wherein the viral vector is an adenovirus.

**12.** The composition of claim 11, wherein the promoter is a liver specific promoter.

**13.** The composition of claim 11, wherein the promoter is a prostate specific promoter.

**14.** The composition of claim 12 or 13 further comprising an interferon alpha secretion leader operatively linked to nucleic acid encoding an interferon alpha polypeptide.

**15.** A pharmaceutical formulation comprising a composition of any of claims 9 through 14.

**16.** A method of providing an interferon alpha polypeptide to a tissue comprising introducing into a tissue of interest a vector comprising a nucleic acid segment encoding an interferon alpha polypeptide, said nucleic acid segment being operatively linked to a promoter having specificity for the tissue of interest, wherein the interferon alpha polypeptide is expressed in the tissue of interest in the patient.

**17.** The method of claim 16, wherein the interferon alpha is interferon alpha 2b.

**18.** The method of claim 17, wherein the viral vector is an adenovirus vector.

**19.** The method of claim 18, wherein the promoter is a liver-specific promoter.

**20.** The method of claim 18, wherein the promoter is a prostate-specific promoter.

**21.** The method of claim 19 and 20, wherein the nucleic acid segment encoding an interferon alpha is operatively linked to nucleic acid encoding an interferon alpha secretion leader.
